# EUROPÄISCHE PATENTSCHRIFT

(11) **EP 1 718 247 B1**
(45) Veröffentlichungstag und Bekanntmachung des Hinweises auf die Patenterteilung: **15.04.2009**
(21) Anmeldenummer: 04715327.5
(22) Anmeldetag: 27.02.2004
(51) Int. Cl.: A61F 2/16

(54) **LINSENAUFNAHME FÜR EINE VORRICHTUNG ZUM EINSETZEN VERFORMBARER INTRAOCULARLINSEN**
LENS HOLDER FOR AN INSERTION DEVICE FOR DEFORMABLE INTRA-OCULAR LENSES
LOGEMENT DE LENTILLE POUR UN DISPOSITIF SERVANT A INSERER DES LENTILLES INTRAOCULAIRES DEFORMABLES

(43) Veröffentlichungstag der Anmeldung: 08.11.2006
(60) Teilanmeldung: 09154434.6
(73) Patentinhaber: Advanced Vision Science, Inc., Goleta, CA 93117 (US)
(72) Erfinder: DEINZER, Klaus, CH-8952 Schlieren (CH); KAMMERLANDER, René Joffre Garden, Block B 2203, Shanghai 200031 (CN)
(74) Vertreter: Secklehner, Günter
(86) Internationale Anmeldenummer: PCT/EP2004/050225
(87) Internationale Veröffentlichungsnummer: WO 2005/082285

(56) Entgegenhaltungen:
- EP-A- 0 402 138
- FR-A- 2 820 633
- US-A- 4 681 102
- US-A- 5 947 975
- US-B1- 6 447 520

## Beschreibung

Die Erfindung betrifft eine Linsenaufnahme für eine Vorrichtung zum Einsetzen verformbarer Intraocularlinsen, mit welcher eine Intraocularlinse aus einem entspannten Zustand in einen elastisch verformten Zustand gebracht wird, um mit Hilfe der Vorrichtung in ein Auge injiziert zu werden, wo sie wieder ihren entspannten Zustand einnimmt, wobei die Linsenaufnahme eine flexible Unterlage mit zwei gegenüberliegenden, verstärkten Randbereichen enthält, welche flexible Unterlage von einer offenen Lage, in der sie eine Intraocularlinse in deren entspanntem Zustand aufzunehmen bestimmt ist in eine geschlossene Lage verformbar ist, in der sie einen Kanal zum Aufnehmen der verformten Intraocularlinse bildet und wobei die Linsenaufnahme in der geschlossenen Lage dazu bestimmt ist, in die Vorrichtung eingesetzt zu werden.

Vorrichtungen zum Einsetzen verformbarer Intraocularlinsen sind bekannt. Ihr Hauptzweck besteht generell darin, den zum Einsetzen einer Intraocularlinse notwendigen Schnitt im Auge möglichst klein halten zu können. Eine Schwierigkeit solcher Vorrichtungen besteht darin, die Intraocularlinse derart in einen elastisch verformten Zustand zu bringen, dass sie in diesem Zu stand durch eine Kanüle in ein Auge injiziert werden kann. Das Patent US4811102 zeigt eine derartige Vorrichtung. Eine Linsenaufnahme weist dabei ein ScJlarnier auf, welches es erlaubt, die Linsenaufnahme von einem offenen Zustand, in dem die Linse eingelegt wird, in einen geschlossenen Zustand zu bringen, in welchem die Linse zusammengefaltet ist. Das sich in der Mitte der Linsenaufnahme befindende Scharnier behindert anfänglich das Verformen der Linse und es kann sogar vorkommen, dass die Linse zu Beginn des Verformungsvorgangs ausweicht und entgegengesetzt zur beabsichtigten Verformungsrichtung gebogen wird. Die Vorrichtung gemäss US5947975 bringt diesbezüglich eine Verbesserung, indem die Linsenaufnahme zwei Scharniere auf weist. Beide der vorangehend erwähnten Vorrichtungen weisen den Nachteil auf, dass es umständlich ist, die Intraocularlinse in der Linsenaufnahme zu platzieren. Ein weiterer Nachteil dieser Vorrichtungen besteht darin, dass mit ihnen die Intraocularlinse praktisch um eine in Längsrichtung der Vorrichtung orientierte Linie gefaltet wird, was zu einer örtlichen Überbeanspruchung der Linse führen kann, wodurch diese unter Umständen dauernd verformt bleibt, insbesondere dann, wenn sie vor der Injektion zu lange in der Vorrichtung verweilte.

Die Patentanmeldung FR2820633A1 zeigt eine Vorrichtung, bei der die zu verformende Linse auf einem flexiblen Teil aufliegt und mit diesem zusammen verformt wird. Mit dieser Vorrichtung lässt sich eine Linse maximal so weit verformen, bis die einander gegenüberliegenden Ränder der Linse aneinander anstossen, wobei die Linse dann im Wesentlichen die Form eines C hat.

Ausgehend von diesem Stand der Technik liegt der Erfindung die Aufgabe zu Grunde, eine Linsenaufnahme vorzuschlagen, bei der eine verformte Intraocularlinse während ihrer Verschiebung in der Linsenaufnahme gerollt wird, derart dass sich ihre gegenüberliegenden Ränder überlappen.

Diese Aufgabe wird erfindungsgemäss dadurch gelöst, dass bei dem in der geschlossenen Lage gebildeten Kanal die Übergänge von der flexiblen Unterlage zu den Randbereichen so ausgebildet sind, dass der Kanal an einem seiner Enden einen schneckenartigen Querschnitt hat.

Durch diese Ausbildung der Linsenaufnahme wird zuverlässig vermieden, dass die gegenüberliegenden Ränder der Linse während des Verschiebens in der Linsenaufnahme aneinander anstossen und so eine weitere Reduktion der Abmessung der verformten Linse verhindern.

Vorteilhaft ist die Linsenaufnahme so gestaltet, dass sie durch eine Biegung der flexiblen Unterlage von der offenen in die geschlossene Lage bringbar ist, wobei die flexible Unterlage und damit auch die mit ihr in Kontakt stehende Intraocularfinse einer zunehmenden Krümmung unterworfen wird. Dies erlaubt ein besonders schonendes Verformen der Intraocularlinse.

Nach einer Ausführungsart ist die flexible Unterlage zwischen der offenen Lage und der geschlossenen Lage elastisch verformbar, so dass sie jeweils wieder ihre Ausgangslage einnimmt, wenn sie von der Verformungskraft entlastet wird. Dabei kann die Linsenaufnahme entweder so gestaltet sein, dass sie in der offenen Lage entspannt ist oder dass sie in der geschlossenen Lage entspannt ist.

Nach einer weiteren Ausführungsart ist beiderseits am Übergang von der flexiblen Unterlage zum jeweiligen Randbereich ein Hinterschnitt zum Halten und Führen der Ränder der Intraocularlinse vorhanden. Diese Randbereiche ermöglichen das genaue Positionieren der Intraocularlinse in der Linsenaufnahme und verhindern zudem das Verrutschen der Intraocularlinse während der Verformung. Vorteil haft weist mindestens einer der hinterschnittenen Randbereiche eine Aussparung auf, damit beim Einlegen der Intraocularlinse deren Rand den Randbereich der Linsenaufnahme ungehindert passieren kann.

In der flexiblen Unterlage kann zudem eine Mulde zur Aufnahme des optischen Teils der Intraocularlinse angeordnet sein, wodurch das genaue Positionieren der Intraocularlinse in der Linsenaufnahme weiter erleichtert wird.

Nach einer weiteren Ausführungsart weist die flexible Unterlage an einem Ende zwischen den Randbereichen eine Verjüngung auf, um eine Führung für einen Stössel zum Transport der verformten Intraocularlinse zu bilden.

Gemäss einer anderen Ausführungsart hat die flexible Unterlage einen sich von der Mitte aus zu beiden Randbereichen hin stetig verändernden Querschnitt. Dadurch lässt sich eine vordefinierte Verformung der Linsenaufnahme erreichen, so dass die Biegelinie der Intraocularlinse optimiert werden kann.

Nach einer weiteren Ausführungsart sind in den Randbereichen der Linsenaufnahme Mittel zum gegenseitigen Verbinden der Randbereiche vorhanden. Diese Verbindungsmittel erlauben es, die Linsenaufnahme in ihrer geschlossenen Lage zu arretieren, was das Einsetzen der Linsenaufnahme mit samt der darin aufgenommenen, verformten Intraocularlinse in eine entsprechende Injektionsvorrichtung erleichtert. Nach einer anderen Ausführungsart sind an den Randbereichen Greifmittel vorhanden, um das Verformen der Linsenaufnahme zu erleichtern.

Nach einer weiteren Ausführungsart wird der in der geschlossenen Lage gebildete Kanal zu einer Seite der Linsenaufnahme hin enger. Dies erlaubt es, die Linse durch ein Verschieben im Kanal weiter zu komprimieren, um sie schliesslich in die genannte Kanüle überzuführen, von der aus sie in ein Auge injiziert werden kann.

Nach einer weiteren Ausführungsart ist mindestens einer der Hinterschnitte zum einen Ende der Linsenaufnahme hin vergrössert, um einen Einlaufabschnitt für eine an der Intraocularlinse angeordnete Haptic zu bilden.

Noch eine Ausführungsart sieht vor, dass an der Linsenaufnahme Rastmittel vorhanden sind, um sie in einem Gehäuse der genannten Vorrichtung zu positionieren und zu halten.

Schliesslich besteht die Linsenaufnahme nach einer weiteren Ausführungsart aus Polypropylen und ist vorzugsweise im Spritzgiessverfahren einstückig hergestellt.

Ausführungsbeispiele der Erfindung werden nachstehend unter Bezugnahme auf die beiliegenden Zeichnungen beispielsweise beschrieben. Es zeigt:
- Figur 1: eine perspektivische Ansicht einer Vorrichtung zum Einsetzen verformbarer Intraocularlinsen mit einem Ausführungsbeispiel einer erfindungsgemässen Linsenaufnahme,
- Figure 2: eine perspektivische Ansicht einer Linsenaufnahme in ihrer offenen Lage nach dem Stand der Technik,
- Figur 3: eine perspektivische Ansicht der Linsenaufnahme nach Figur 2 in ihrer geschlossenen Lage,
- Figur 4: eine perspektivische Ansicht einer Ausführungsart der Linsenaufnahme in ihrer offenen Lage,
- Figur 5: eine perspektivische Ansicht der Linsenaufnahme nach Figur 4 in ihrer geschlossenen Lage und
- Figur 6: eine perspektivische Ansicht einer Ausführungsart der Linsenaufnahme in ihrer geschlossenen Lage, eingebaut in einer Vorrichtung.

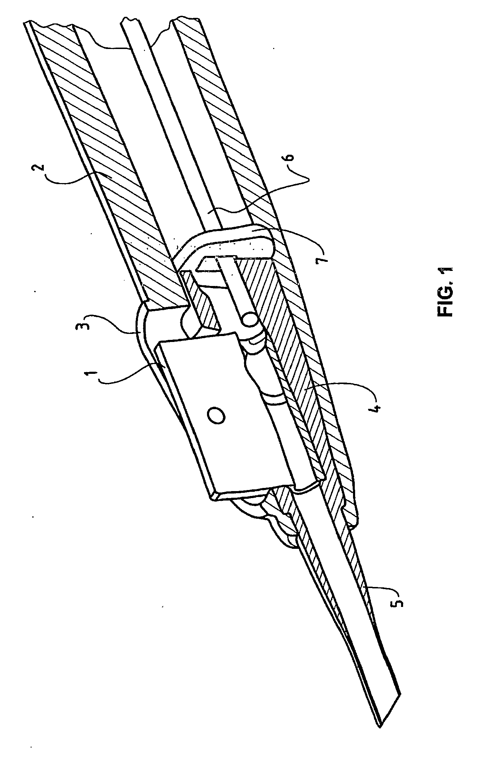

Figur 1 zeigt einen Ausschnitt aus einer Vorrichtung zum Einsetzen verformbarer Intraocularlinsen mit einer in der Vorrichtung eingesetzten Linsenaufnahme 1. Die Vorrichtung weist ein längliches Gehäuse 2 auf, in dessen Seitenwand eine längliche Öffnung 3 vorgesehen ist, die zum Einsetzen der Linsenaufnahme 1 dient. Im vorliegenden Beispiel ist die Linsenaufnahme 1 in einem Lagerteil 4 gehalten, wobei dieser Lagerteil 4 mit einer Kanüle 5 einstückig verbunden ist. Beim Gebrauch der Vorrichtung wird die Linsenaufnahme 1 mit einer darin im verformten Zustand aufgenommenen Intraocularlinse durch die Öffnung 3 in die Vorrichtung eingesetzt. Dann wird die Intraocularlinse durch einen Stössel 6 aus der Linsenaufnahme 1 in die Kanüle 5 vorgeschoben. Anschliessend wird die Kanüle 5 durch einen kleinen Einschnitt in das Auge eines Patienten eingesetzt und die Intraocularlinse wird durch den Stössel 6 aus der Kanüle 5 in das Auge geschoben. Ein im Gehäuse 2 angeordneter Führungsteil 7 sorgt dafür, dass der Stössel 6 die Intraocularlinse präzise erfasst.

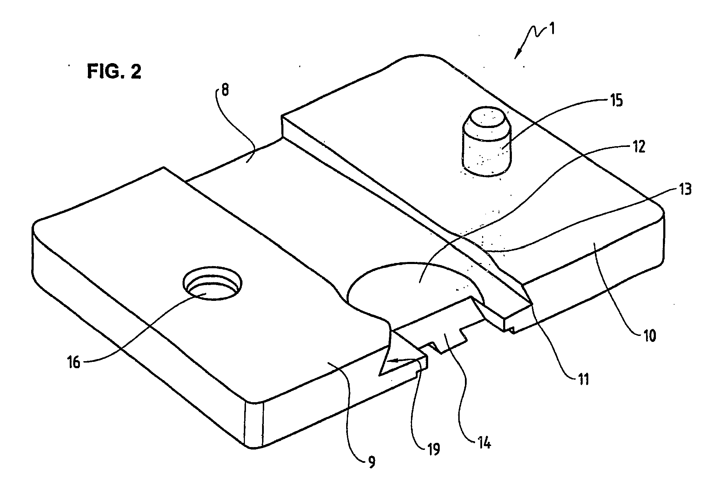

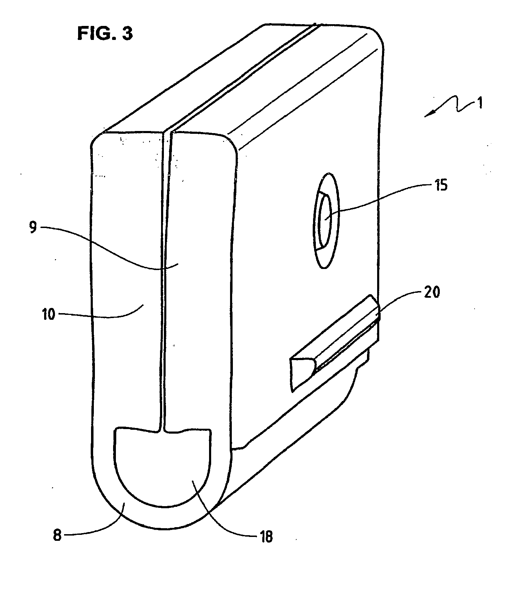

Figur 2 zeigt eine perspektivische Ansicht einer Linsenaufnahme 1 in ihrer zur Aufnahme einer Intraocularlinse (nicht dargestellt) bereiten, offenen Lage. Die Linsenaufnahme 1 weist eine flexible Unterlage 8 auf, welche in dieser Lage im Wesentlichen eben ist. Beiderseits der flexiblen Unterlage 8 weist die Linsenaufnahme 1 Randbereiche 9 und 10 auf, die dicker und dadurch steifer sind als die flexible Unterlage 8. Am Über gang zwischen der flexiblen Unterlage zum Randbereich ist jeweils ein Hinterschnitt 11 vorhanden, der es erlaubt, die Ränder der einzulegenden Intraocularlinse während des Verformungsvorgangs zu halten und bei der Verschiebung in die Kanüle 5 zu führen. Um das Einlegen der Intraocularlinse zu erleichtern, ist in beiden Randbereichen jeweils eine Aussparung 13 angeordnet, die jedoch nicht bis zum Grund des Hinterschnitts 11 reicht. Eine in der flexiblen Unterlage 8 angeordnete Mulde 12 kann das Platzieren der Intraocularlinse weiter erleichtern. Bekannte Intraocularlinsen weisen zwei so genannte Haptics auf. Dies sind kleine Bügel, welche die Linse in der Linsentasche im Auge zentrieren. Um solche Intraocularlinsen mit der Linsenaufnahme einzusetzen, weist die Linsenaufnahme 1 im Bereich eines der Hinterschnitte 11 eine Erweiterung 19 auf, welche eine Haptic aufnimmt und verhindert, dass dieser später beim Verschieben der Intraocularlinse durch den Stössei 6 an der Linsenaufnahme 1 hängen bleibt. Wenn eine Intraocularlinse in der soeben beschriebenen Art in der Linsenaufnahme 1 platziert wurde, wird diese an ihren beiden Randbereichen 9 und 10 ergriffen und mitsamt der auf der flexiblen Unterlage 8 aufliegenden Intraocularlinse verformt, bis sie die in Figur 3 dargestellte Lage einnimmt. Eine Verjüngung 14 ist an einer Seite der flexiblen Unterlage 8 angeordnet und hat den Zweck, nach dem Verformen und dem Einsetzen in die Vorrichtung den Stössel 6 derart zu führen, dass er genau den Rand der verformten Intraocularlinse trifft, wenn diese aus der Linsenaufnahme 1 in die Kanüle 5 geschoben werden soll.

Figur 3 zeigt eine perspektivische Ansicht der Linsenaufnahme 1 gemäss Figur 2 in ihrer geschlossenen Lage. Deutlich ist ein Kanal 18 zu sehen, der durch die gebogene flexible Unterlage 8 und die Randbereiche 9 und 10 begrenzt ist. Damit die Linsenaufnahme 1 bis zum Einsetzen in die Vorrichtung in ihrer in Figur 3 dargestellten geschlossenen Lage verbleibt, sind Verbindungsmittel vorgesehen, die im vorliegenden Beispiel aus Zapfen 15 bestehen, die am Randbereich 10 vorhanden sind und in Öffnungen 16 eingreifen, die im Randbereich 9 angeordnet sind. An mindestens einer Seite der geschlossenen Linsenaufnahme sind Rastmittel, beispielsweise in der Form einer Rippe 20 angeordnet, welche dazu bestimmt sind, die Linsenaufnahme 1 im Gehäuse 2 der Vorrichtung genau zu positionieren und zu arretieren.

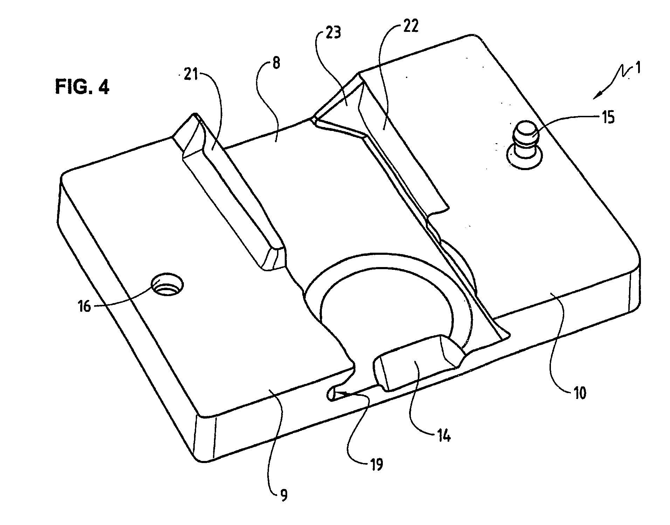

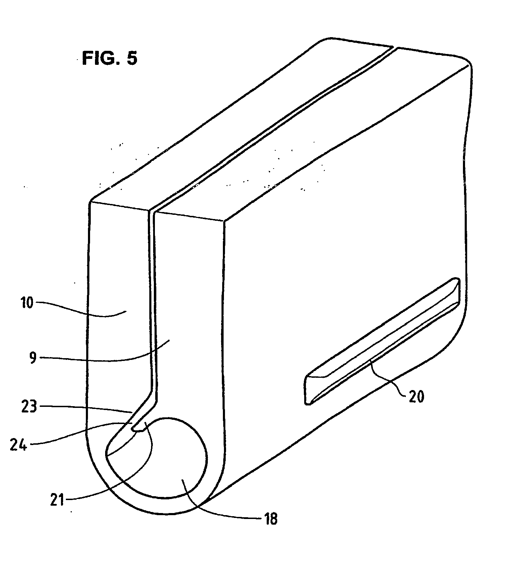

Die Figuren 4 und 5 zeigen eine Ausführungsart der Linsenaufnahme 1, welche speziell zum Injizieren von relativ grossen Intraocularlinsen vorgesehen ist. Figur 4 zeigt dabei die geöffnete Lage der Linsenaufnahme 1 und Figur 5 die geschlossene Lage. Für gleiche Teile wie in den Figuren 1 bis 3 sind gleiche Bezugszeichen verwendet. Die Linsenaufnahme 1 nach den Figuren 4 und 5 unterscheidet sich von jener nach den Figuren 2 und 3 insbesondere durch eine Erhebung 21, die im Randbereich 9 angrenzend an die flexible Unterlage 8 angeordnet ist. Im gegenüberliegenden Randbereich 10 ist eine Ausnehmung 22 vorgesehen, in welcher die Erhebung 21 in der geschlossenen Lage der Linsenaufnahme 1 Platz findet. In Figur 5 ist deutlich zu sehen, dass der Kanal 18 an der Austrittsseite durch die Erhebung 21 und die Ausnehmung 22 einen schneckenartigen Querschnitt erhält. Zwischen der Erhebung 21 und der Ausnehmung 22 ist dabei ein Spalt 24 vorhanden, der es erlaubt, eine grosse Intraocularlinse derart zu verformen, dass sich deren gegenüberliegende Ränder überschneiden, wodurch die Intraocularlinse die Linsenaufnahme 1 in einem aufgerollten Zustand verlässt. Um die derart aufgerollte Intraocularlinse in die Kanüle 5 überzuführen, ist im Randbereich 10 ein Leitteil 23 angeordnet. Bei dieser Ausführungsart sind die Zapfen 15 hinterschnitten, um in den Öffnungen 16 eine Rastwirkung zu erzielen.

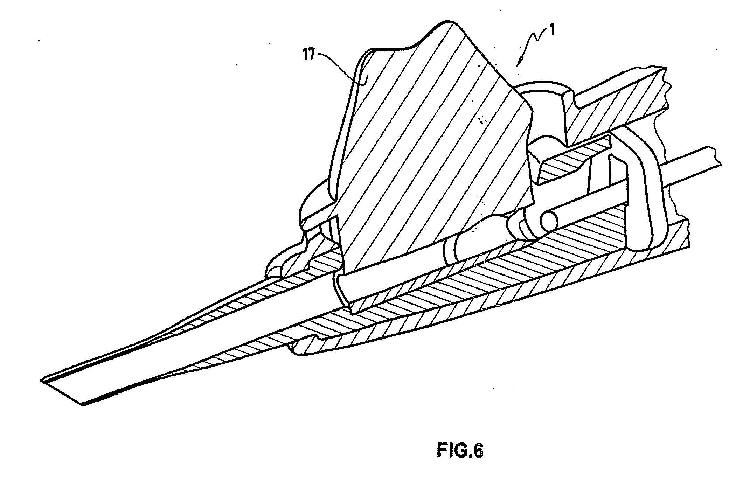

Bei der Ausführungsart nach Figur 6 sind an den Randbereichen 9 und 10 flügelartige Griffe 17 angeformt, damit sich die betreffende Linsenaufnahme besser ergreifen, verformen und halten lässt.

Gemäss einer weiteren, nicht zeichnerisch dargestellten Ausführungsart der Erfindung kann die flexible Unterlage eine Schlaufe bilden, welche eine Intraocularlinse in ihrem entspannten Zustand aufnimmt und dann zusammengezogen wird, beispielsweise etwa so, wie dies bei Kabelbindern der Fall ist.

## Patentansprüche

1. Linsenaufnahme (1) für eine Vorrichtung zum Einsetzen verformbarer Intraocularlinsen, mit welcher eine Intraocularlinse aus einem entspannten Zustand in einen elastisch verformten Zustand gebracht wird, um mit Hilfe der Vorrichtung in ein Auge injiziert zu werden, wo sie wieder ihren entspannten Zustand einnimmt, wobei die Linsenaufnahme (1) eine flexible Unterlage (8) mit zwei gegenüberliegenden, verstärkten Randbereichen (9, 10) enthält, welcher flexible Unterlage (8) von einer offenen Lage, in der sie eine lntraocularlinse in deren entspanntem Zustand aufzunehmen bestimmt ist in eine geschlossene Lage verformbar ist, in der sie einen Kanal (18) zum Aufnehmen der verformten Intraocularlinse bildet und wobei die Linsenaufnahme (1) in der geschlossenen Lage dazu bestimmt ist, in die Vorrichtung eingesetzt zu werden, **dadurch gekennzeichnet, dass** bei dem in der geschlossenen Lage gebildeten Kanal (18) die Übergänge von der flexiblen Unterlage (8) zu den Randbereichen (9, 10) so ausgebildet sind, dass der Kanal an einem seiner Enden einen schneckenartigen Querschnitt hat.

2. Linsenaufnahme nach Anspruch 1, **dadurch gekennzeichnet, dass** sie derart ausgebildet ist, dass sie durch eine Biegung der flexiblen Unterlage (8) von der offenen in die geschlossene Lage bringbar ist, wobei die flexible Unterlage (8) und damit auch die mit ihr in Kontakt stehende Intraocularlinse einer zunehmenden Krümmung unterworfen wird.

3. Linsenaufnahme nach einem der vorangehenden Ansprüche, da durch **gekennzeichnet**, dass die flexible Unterlage (8) zwischen der offenen Lage und der geschlossenen Lage elastisch verform bar ist, wobei sie in der offenen Lage entspannt ist.

4. Linsenaufnahme nach einem der Ansprüche 1 bis 2, **dadurch gekennzeichnet, dass** die flexible Unterlage (8) zwischen der offenen Lage und der geschlossenen Lage elastisch verformbar ist, wobei sie in der geschlossenen Lage entspannt ist.

5. Linsenaufnahme nach einem der vorangehenden Ansprüche, **dadurch gekennzeichnet, dass** beiderseits am Übergang von der flexiblen Unterlage (8) zum jeweiligen Randbereich (9, 10) ein Hinterschnitt (11) zum Halten und Führen der Ränder der Intraocularlinse vorhanden ist.

6. Linsenaufnahme .nach Anspruch 5, **dadurch gekennzeichnet, dass** mindestens einer der hinterschnittenen Randbereiche (9, 10) eine Aussparung (13) aufweist, damit beim Einlegen der Intraocularlinse deren Rand den Randbereich der Linsenaufnahme ungehindert passieren kann.

7. Linsenaufnahme nach einem der vorangehenden Ansprüche, **dadurch gekennzeichnet, dass** in der flexiblen Unterlage eine Mulde (12) zur Aufnahme des optischen Teils der Intraocularlinse angeordnet ist.

8. Linsenaufnahme nach einem der vorangehenden Ansprüche, **dadurch gekennzeichnet, dass** die flexible Unterlage (8) an einem Ende zwischen den Randbereichen (9, 10) eine Verjüngung (14) aufweist, um eine Führung für einen Stössel (6) zum Transport der verformten Intraocularlinse zu bilden.

9. Linsenaufnahme nach einem der vorangehenden Ansprüche, **dadurch gekennzeichnet, dass** die flexible Unterlage (8) einen sich von der Mitte aus zu beiden Randbereichen (9, 10) hin stetig verändernden Querschnitt hat.

10. Linsenaufnahme nach einem der vorangehenden Ansprüche, **dadurch gekennzeichnet, dass** in den Randbereichen (9, 10) Mittel (15, 16) zum gegenseitigen Verbinden der Randbereiche vorhanden sind.

11. Linsenaufnahme nach einem der vorangehenden Ansprüche, **dadurch gekennzeichnet, dass** an den Randbereichen Greifmittel (17) vorhanden sind, um das Verformen der flexiblen Unterlage (8) zu erleichtern.

12. Linsenaufnahme nach einem der vorangehenden Ansprüche, **dadurch gekennzeichnet, dass** der in der geschlossenen Lage gebildete Kanal (18) zu einer Seite der Linsenaufnahme hin enger wird.

13. Linsenaufnahme nach einem der Ansprüche 5 bis 6, **dadurch gekennzeichnet, dass** mindestens einer der der Hinterschnitte (11) zum einen Ende der Linsenaufnahme hin vergrössert ist, um einen Einlaufabschnitt (19) für eine an der Intraocularlinse angeordnete Haptic zu bilden.

14. Linsenaufnahme nach einem der vorangehenden Ansprüche, **dadurch gekennzeichnet, dass** an ihr Rastmittel (20) vorhanden sind, um die Linsenaufnahme in einem Gehäuse (2) der genannten Vorrichtung zu positionieren und zu halten.

15. Linsenaufnahme nach einem der vorangehenden Ansprüche, **dadurch gekennzeichnet, dass** sie aus Polypropylen besteht und vorzugsweise im Spritzgiessverfahren einstückig hergestellt ist.

## Claims

1. Lens holder (1) for a device for inserting deformable intra-ocular lenses, by means of which an intra-ocular lens can be transferred from a relaxed state into an elastically deformed state so that it can be injected with the aid of the device into an eye where it resumes its relaxed state, which lens holder (1) contains a flexible backing support (8) with two oppositely lying, reinforced peripheral regions (9, 10), and the flexible backing support (8) can be deformed from an open position, in which it is designed to accommodate an intra-ocular lens in its relaxed state, into a closed position, in which it forms a passage (18) for accommodating the deformed intra-ocular lens, the lens holder (1) being designed to be inserted in the device when it is in the closed position, **characterised in that** the passage (18) formed in the closed position is such that the passage has a snail-type cross section at one of its ends in the regions of the passage where the flexible backing support (8) merges into the peripheral regions (9, 10).

2. Lens holder as claimed in claim 1, **characterised in that** it is designed so that it can be transferred from the open into the closed position by bending the flexible backing support (8), and an increasing curvature is imparted to the flexible backing support (8) and hence also to the intra-ocular lens in contact with it.

3. Lens holder as claimed in one of the preceding claims, **characterised in that** the flexible backing support (8) can be elastically deformed between the open position and the closed position and is relaxed in the open position.

4. Lens holder as claimed in one of claims 1 to 2, **characterised in that** the flexible backing support (8) can be elastically deformed between the open position and the closed position and is relaxed in the closed position.

5. Lens holder as claimed in one of the preceding claims, **characterised in that** an undercut (11) is provided on either side at the transition from the flexible backing support (8) to the respective peripheral regions (9, 10) for retaining and guiding the edges of the intra-ocular lens.

6. Lens holder as claimed in claim 5, **characterised in that** at least one of the undercut peripheral regions (9, 10) has a recess (13) so that when the intra-ocular lens is inserted, its edge is able to move past the peripheral region of the lens holder unhindered.

7. Lens holder as claimed in one of the preceding claims, **characterised in that** a depression (12) is provided in the flexible backing support for accommodating the optical part of the intra-ocular lens.

8. Lens holder as claimed in one of the preceding claims, **characterised in that** the flexible backing support (8) has a tapered region (14) between the peripheral regions (9, 10) at one end, serving as a guide for a push rod (6) for conveying the deformed intra-ocular lens.

9. Lens holder as claimed in one of the preceding claims, **characterised in that** the flexible backing support (8) has a constantly changing cross-section from the middle to the two peripheral regions (9, 10).

10. Lens holder as claimed in one of the preceding claims, **characterised in that** means (15, 16) are provided in the peripheral regions (9, 10) for mutually connecting the peripheral regions.

11. Lens holder as claimed in one of the preceding claims, **characterised in that** gripping means (17) are provided at the peripheral regions to make it easier to deform the flexible backing support (8).

12. Lens holder as claimed in one of the preceding claims, **characterised in that** the passage (18) formed in the closed position becomes narrower towards one end of the lens holder.

13. Lens holder as claimed in one of claims 5 to 6, **characterised in that** at least one of the undercuts (11) becomes larger towards one end of the lens holder in order to form an inlet portion (19) for a haptic means provided on the intra-ocular lens.

14. Lens holder as claimed in one of the preceding claims, **characterised in that** catch means (20) are provided on it in order to position and retain the lens holder in a housing (2) of said device.

15. Lens holder as claimed in one of the preceding claims, **characterised in that** it is made from polypropylene and is preferably manufactured integrally by an injection moulding process.

## Revendications

1. Logement de lentille (1) pour un dispositif servant à insérer des lentilles intraocculaires déformables, au moyen duquel une lentille intraocculaire est amenée d'un état détendu dans un état élastiquement déformé, pour être injectée à l'aide du dispositif dans un oeil, où elle reprend à nouveau son état détendu, où le logement de lentille (1) contient un support flexible (8) avec deux zones de bord renforcées opposées (9, 10), ledit support flexible (8) pouvant être déformé à partir d'une position ouverte, dans laquelle il est destiné à recevoir une lentille intraocculaire dans son état détendu, en une position fermée, dans laquelle il forme un canal (18) pour la réception de la lentille intraocculaire déformée, et où le logement de lentille (1), en position fermée, est destiné à être placé dans le dispositif, **caractérisé en ce que** dans le canal (18) formé en position fermée, les transitions du support flexible (8) aux zones de bord (9, 10) sont réalisées de telle sorte que le canal possède à l'une de ses extrémités une section transversale hélicoïdale.

2. Logement de lentille selon la revendication 1, **caractérisé en ce qu'**il est réalisé de telle sorte qu'il peut être amené par une flexion du support flexible (8) de la position ouverte dans la position fermée, où le support flexible (8) et donc également la lentille intraocculaire en contact avec celui-ci sont soumis à une courbure croissante.

3. Logement de lentille selon l'une des revendications précédentes, **caractérisé en ce que** le support flexible (8) est déformable élastiquement entre la position ouverte et la position fermée, où il est détendu en position ouverte.

4. Logement de lentille selon l'une des revendications 1 à 2, **caractérisé en ce que** le support flexible (8) est déformable élastiquement entre la position ouverte et la position fermée, où il est détendu en position fermée.

5. Logement de lentille selon l'une des revendications précédentes, **caractérisé en ce qu'**il est réalisé, des deux côtés à la transition du support flexible (8) à la zone de bord respective (9, 10), une contre-dépouille (11) pour tenir et guider les bords de la lentille intraocculaire.

6. Logement de lentille selon la revendication 5, **caractérisé en ce qu'**au moins une des zones de bord contre-dépouillées (9,10) présente un évidement (13) pour que lors de l'insertion de la lentille intraocculaire, son bord peut passer, sans être gêné, devant la zone de bord du logement de lentille.

7. Logement de lentille selon l'une des revendications précédentes, **caractérisé en ce qu'**est disposé dans le support flexible un creux (12) pour la réception de la partie optique de la lentille intraocculaire.

8. Logement de lentille selon l'une des revendications précédentes, **caractérisé en ce que** le support flexible (8) présente à une extrémité, entre les zones de bord (9, 10), un retrécissement (14) pour former un guidage pour un poussoir (6) pour le transport de la lentille intraocculaire déformée.

9. Logement de lentille selon l'une des revendications précédentes, **caractérisé en ce que** le support flexible (8) possède une section transversale se modifiant en continu du milieu vers les deux zones de bord (9, 10).

10. Logement de lentille selon l'une des revendications précédentes, **caractérisé en ce que** sont prévus dans les zones de bord (9, 10) des moyens (15, 16) pour relier mutuellement les zones de bord.

11. Logement de lentille selon l'une des revendications précédentes, **caractérisé en ce que** sont prévus aux zones de bord des moyens de préhension (17) pour faciliter la déformation du support flexible (8).

12. Logement de lentille selon l'une des revendications précédentes, **caractérisé en ce que** le canal (18) formé dans la position fermée devient plus étroit vers un côté du logement de lentille.

13. Logement de lentille selon l'une des revendications 5 à 6, **caractérisé en ce qu'**au moins l'une des contre-dépouilles (11) est agrandie vers une extrémité du logement de lentille pour former une section d'entrée (19) pour un haptique disposé à la lentille intraocculaire.

14. Logement de lentille selon l'une des revendications précédentes, **caractérisé en ce que** des moyens d'enclenchement (20) sont réalisés à celui-ci pour positionner et tenir le logement de lentille dans un boîtier (2) du dispositif précité.

15. Logement de lentille selon l'une des revendications précédentes, **caractérisé en ce qu'**il est réalisé en polypropylène et est réalisé de préférence en une pièce par un procédé de moulage par injection.
